# DEMANDE DE BREVET EUROPEEN

(11) **EP 3 778 877 A1**
(43) Date de publication de la demande: **17.02.2021**
(21) Numéro de dépôt: 20188746.0
(22) Date de dépôt: 27.05.2016
(51) Int. Cl.: C12N 5/078, A61L 2/00

(54) **PROCÉDÉ DE STÉRILISATION D'UN LYSAT PLAQUETTAIRE**

(30) Priorité: 29.05.2015 FR 1554883
(62) Demande divisionnaire de: 16731231.3
(71) Demandeur: Maco Pharma, 59420 Mouvaux (FR)
(72) Inventeur: DELORME, Bruno, 59700 Marcq-en-Baroeul (FR); VIAU, Sabrina, 59290 Wasquehal (FR); GOUDALIEZ, Francis, 59155 Faches-Thumesnil (FR)
(74) Mandataire: Sayettat, Julien Christian

(57) **Abrégé**

Procédé de stérilisation d'un lysat plaquettaire à l'état liquide comprenant au moins les facteurs de croissance endogènes TGF-beta1, EGF, PDGF-AB, IGF-1, VEGF et bFGF, ledit procédé comprenant la congélation dudit lysat plaquettaire liquide afin d'obtenir un lysat plaquettaire congelé et l'irradiation par un rayonnement ionisant dudit lysat plaquettaire congelé afin d'obtenir un lysat plaquettaire stérilisé, ladite irradiation étant agencée de sorte à conserver au moins 80% de la concentration de l'un au moins des facteurs de croissance endogènes choisi dans le groupe constitué par le TGF-beta1, l'EGF, le PDGF-AB, l'IGF-1 et le VEGF.

## Description

L'invention concerne un procédé de stérilisation d'un lysat plaquettaire ainsi qu'un lysat plaquettaire stérilisé obtenu par un tel procédé et un procédé de culture de cellules utilisant un tel lysat plaquettaire stérilisé.

L'invention s'applique au domaine des produits dérivés des plaquettes sanguines, et notamment au domaine de la culture cellulaire pour cultiver les cellules à usage thérapeutique, plus particulièrement les cellules souches mésenchymateuses.

Pour cultiver des cellules animales *in vitro,* on utilise classiquement des milieux de base de type RPMI (Roswell Park Memorial Institute), MEM (Modified Eagle Medium) ou DMEM (Dulbecco Modified Eagle Medium) comprenant essentiellement des sels minéraux, du glucose, des acides aminés, des vitamines et des bases azotées. Ces milieux sont généralement complémentés extemporanément avec des antibiotiques pour prévenir la contamination bactérienne, de la L-glutamine, un acide aminé instable, et entre 1,5 et 10% de sérum de veau fœtal comme complément nutritif.

Cependant, le sérum de veau fœtal est un vecteur potentiel d'agents pathogènes xénogènes. En particulier, lorsque le sérum est d'origine bovine, le risque de contamination par les prions ou les virus n'est pas nul.

Pour remédier à ces inconvénients, il a été proposé de remplacer le sérum par du lysat plaquettaire humain qui présente l'avantage de comprendre une quantité importante de facteurs de croissance d'origine humaine tels que par exemple le facteur de croissance transformant bêta1 (TGF-beta1, Transforming Growth Factor-beta1), le facteur de croissance épidermique (EGF, Epidermal Growth Factor), le facteur de croissance dérivé des plaquettes AB (PDGF-AB, Platelet-Derived Growth Factor-AB), le facteur de croissance 1 ressemblant à l'insuline (IGF-1, Insulin-like Growth Factor-1), le facteur de croissance de l'endothélium vasculaire (VEGF, Vascular Endothelial Growth Factor) et le facteur de croissance des fibroblastes 2 (FGF-2, Fibroblast Growth Factor 2), aussi appelé facteur de croissance des fibroblastes basique (bFGF, Basic Fibroblast Growth Factor).

Il a ainsi été montré un meilleur taux de prolifération des cellules souches mésenchymateuses cultivées dans un milieu de base complémenté avec du lysat plaquettaire humain par rapport à un milieu de référence comprenant le même milieu de base complémenté avec du sérum de veau fœtal (SVF) et additionné de 1 ng/ml de FGF-2 (Azouna, N. Ben, et al. "Phenotypical and functional characteristics of mesenchymal stem cells from bone marrow: comparison of culture using different media supplemented with human platelet lysate or fetal bovine serum." Stem Cell Res Ther 3.1 (2012): 6).

Cependant, le lysat plaquettaire étant d'origine humaine, il présente lui aussi un risque de contamination pathogène qu'il est nécessaire de prendre en compte et de limiter au maximum. En effet, les lysats plaquettaires peuvent être des vecteurs potentiels de virus, bactéries ou protozoaires.

Ainsi, il existe un réel besoin de développer des lysats plaquettaires plus sécurisés pour la culture cellulaire.

Le document WO 2013/042095 et l'article de S. Castiglia (Castiglia, Sara, et al. "Inactivated human platelet lysate with psoralen: a new perspective for mesenchymal stromal cell production in Good Manufacturing Practice conditions." Cytotherapy 16.6 (2014): 750-763) divulguent un lysat plaquettaire obtenu à partir de concentrés de plaquettes issus de buffy coat qui ont subi une inactivation virale par un rayonnement ultraviolet en présence de psoralène. Cette technique d'inactivation virale présente cependant l'inconvénient de devoir utiliser une molécule supplémentaire qui doit ensuite être éliminée du produit traité.

Le document WO 2010/033605 propose de faire passer le lysat plaquettaire au travers d'un filtre de 0,45 µm ou 0,22 µm. Cependant, de tels filtres ne permettent pas d'éliminer les virus de petites tailles éventuellement présents.

Le document WO 2011/148326 propose de préparer un lysat plaquettaire inactivé viralement en traitant un concentré de plaquettes au solvant/détergent. Cependant, la méthode chromatographique utilisée pour éliminer le solvant/détergent du lysat ainsi obtenu, conduit à éliminer notamment les facteurs de croissance VEGF et PDGF.

En variante décrite dans le document US 2012/0156306, le lysat plaquettaire traité par solvant/détergent subit ensuite une deuxième méthode d'inactivation virale choisie parmi la pasteurisation, la nano filtration, le traitement à faible pH, l'irradiation aux ultraviolets ou encore le traitement au thiocyanate de sodium.

Dans le cas de protéines, le document WO 01/70279 enseigne que l'irradiation par rayonnement gamma doit être réalisée sur des protéines sans solvant résiduel, c'est-à-dire sous une forme lyophilisée, et/ou en présence d'un stabilisateur afin de ne pas les dénaturer.

Cependant, la lyophilisation est un procédé discontinu qui se traduit par de multiples manipulations et des durées de traitement relativement importantes qui augmentent ainsi les coûts liés à l'utilisation de tels procédés. L'ajout de stabilisateur constitue également une étape et un composé supplémentaires dans le traitement du produit dont il serait avantageux de se passer.

De plus, même sous forme lyophilisée, certains facteurs de croissance ne résistent pas à l'irradiation gamma. Par exemple, le document WO 00/33893 montre une perte d'activité de plus de 90% du facteur de croissance PDGF lyophilisé stérilisé par irradiation gamma par rapport au PDGF lyophilisé non stérilisé.

Enfin, le document WO 2014/076200 décrit des compléments de culture basés sur des fractions de plasma riches ou pauvres en plaquettes. Il est indiqué que de tels compléments de culture peuvent être congelés, lyophilisés, stérilisés par irradiation gamma à une dose de 1kGy et stockés à -20°C. Cette dose d'irradiation relativement faible n'est pas suffisante pour inactiver les virus et bactéries.

De manière inattendue et contrairement à l'enseignement des documents antérieurs, il a été observé qu'un lysat plaquettaire ayant subi, dans un état congelé, une forte dose d'irradiation par rayonnement gamma conserve une bonne activité biologique sans nécessiter de lyophilisation ni de stabilisateur.

En outre, alors que l'ajout de bFGF exogène à du lysat plaquettaire non irradié n'a aucun effet sur le taux de prolifération cellulaire (Pérez-Ilzarbe, Maitane, et al. "Comparison of ex vivo expansion culture conditions of mesenchymal stem cells for human cell therapy." Transfusion 49.9 (2009): 1901-1910), le lysat plaquettaire irradié à l'état congelé par rayonnement gamma présente la propriété nouvelle d'augmenter le taux de prolifération cellulaire en présence de bFGF exogène.

Ainsi, selon un premier aspect, l'invention propose un procédé de stérilisation d'un lysat plaquettaire à l'état liquide comprenant au moins les facteurs de croissance endogènes TGF-beta1, EGF, PDGF-AB, IGF-1, VEGF et bFGF, ledit procédé comprenant :
- la congélation dudit lysat plaquettaire liquide afin d'obtenir un lysat plaquettaire congelé,
- l'irradiation par un rayonnement ionisant dudit lysat plaquettaire congelé afin d'obtenir un lysat plaquettaire stérilisé, ladite irradiation étant agencée de sorte à conserver au moins 80% de la concentration de l'un au moins des facteurs de croissance endogènes choisi dans le groupe constitué par le TGF-beta1, l'EGF, le PDGF-AB, l'IGF-1 et le VEGF.

Selon un deuxième aspect, l'invention concerne le lysat plaquettaire stérilisé obtenu par le procédé selon le premier aspect de l'invention.

Selon un troisième aspect, l'invention propose un procédé pour la culture de cellules, notamment de cellules souches mésenchymateuses, comprenant la mise en contact desdites cellules avec une composition nutritive comprenant un milieu de base et un lysat plaquettaire stérilisé selon le deuxième aspect de l'invention.

D'autres objets et avantages apparaîtront au cours de la description qui suit.

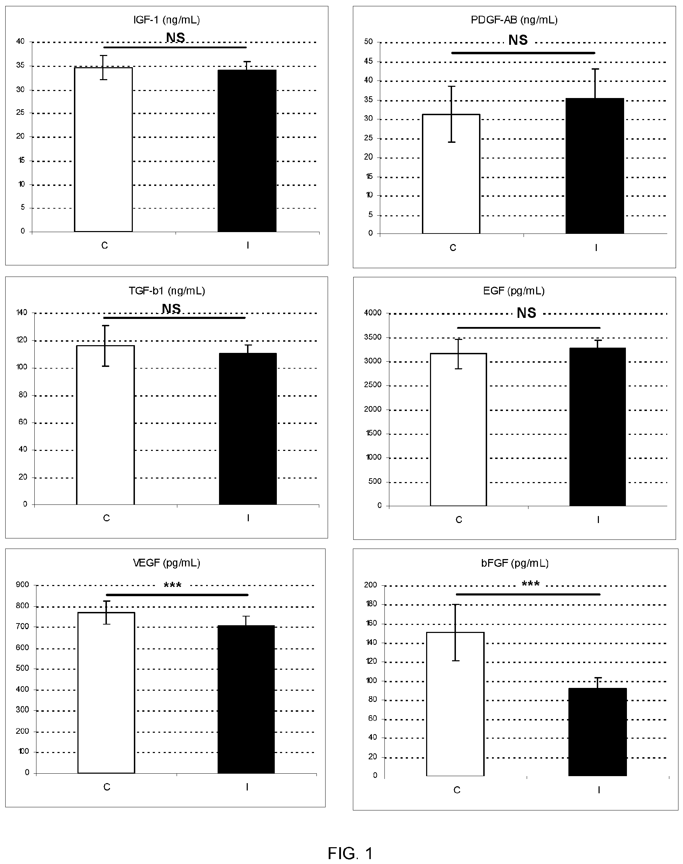

La figure 1 représente les moyennes des concentrations en facteurs de croissance IGF-1, PDGF-AB, TGF-beta1, EGF, VEGF et bFGF dans des lysats plaquettaires non irradiés (C) et dans lysats plaquettaires stérilisés par mise en œuvre du procédé de l'invention (I).

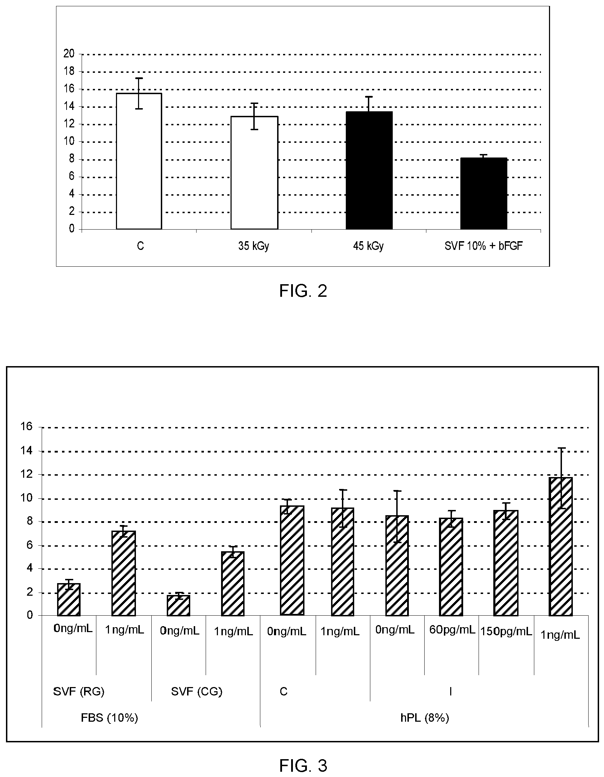

La figure 2 montre la moyenne des taux d'amplification de cellules souches mésenchymateuses cultivées en présence de lysats plaquettaires non irradiés (C), de lysats plaquettaires stérilisés à l'état congelé par irradiation gamma à 35 kGy, de lysats plaquettaires stérilisés à l'état congelé par irradiation gamma à 45 kGy et de sérum de veau fœtal complémenté en bFGF.
La figure 3 représente les moyennes des taux d'amplification de cellules souches mésenchymateuses cultivées en présence de lysats plaquettaires non irradiés (C), de lysats plaquettaires stérilisés à l'état congelé par irradiation gamma à 35 kGy, de lysats plaquettaires stérilisés à l'état congelé par irradiation gamma à 45 kGy et de sérum de veau fœtal (SVF (RG) et SVF (CG)), avec des concentrations variables de bFGF exogène ajouté.

L'invention concerne un procédé de stérilisation d'un lysat plaquettaire en vue d'obtenir un lysat plaquettaire stérilisé.

Par procédé de stérilisation, on désigne un procédé de réduction et/ou d'inactivation des pathogènes comprenant les virus, les bactéries, les champignons et les spores bactériennes.

Par lysat plaquettaire, on désigne le produit de la lyse de plaquettes, c'est-à-dire le produit obtenu après désintégration de la membrane cellulaire qui conduit à la libération des molécules (facteurs de croissance, cytokines) normalement contenues à l'intérieur des plaquettes.

Ainsi, le lysat plaquettaire comprend notamment un concentré de facteurs de croissance incluant l'IGF-1, PDGF-AB, TGF-beta1, EGF, VEGF et bFGF. D'autres facteurs de croissance se trouvant dans le lysat plaquettaire sont notamment le facteur de croissance du tissu conjonctif (CTGF, Connective Tissue Growth Factor) et le facteur dérivé des cellules stromales de type 1-alpha (SDF-1alpha, Stromal Cell-Derived Factor-1alpha). Ces facteurs de croissance sont dits endogènes.

Par substance endogène, on désigne toute substance produite par les plaquettes ou comprise dans la suspension de plaquettes initiale utilisées pour préparer le lysat plaquettaire, par opposition à une substance exogène introduite dans le lysat plaquettaire ou dans la suspension de plaquettes initiale.

La lyse des plaquettes est par exemple réalisée par un ou plusieurs cycles de congélation/décongélation, par l'utilisation d'ultra-sons ou par un traitement au solvant/détergent.

Dans le procédé de stérilisation décrit, le lysat plaquettaire provient de plaquettes animales, notamment humaines, issues d'un procédé d'aphérèse ou préparées à partir d'un don de sang.

Selon un premier aspect de l'invention, le procédé de stérilisation d'un lysat plaquettaire est réalisé à partir d'un lysat plaquettaire à l'état liquide, ledit lysat plaquettaire comprenant au moins les facteurs de croissance endogènes IGF-1, PDGF-AB, TGF-beta1, EGF, VEGF et bFGF, ledit procédé comprenant :
- la congélation dudit lysat plaquettaire liquide afin d'obtenir un lysat plaquettaire congelé,
- l'irradiation par un rayonnement ionisant dudit lysat plaquettaire congelé afin d'obtenir un lysat plaquettaire stérilisé, ladite irradiation étant agencée de sorte à conserver au moins 80% de la concentration de l'un au moins des facteurs de croissance endogènes choisi dans le groupe constitué par le TGF-beta1, l'EGF, le PDGF-AB, l'IGF-1 et le VEGF.

Le lysat plaquettaire à l'état liquide est obtenu à partir d'une suspension de plaquettes. La suspension de plaquettes est notamment un concentré de plaquettes ou un mélange de concentrés de plaquettes, une couche leuco-plaquettaire, aussi appelée buffy coat, ou un mélange de couches leuco-plaquettaires, un plasma riche en plaquettes ou un mélange de plasma riche en plaquettes.

Plus particulièrement, la suspension de plaquettes est un concentré plaquettaire issu d'aphérèse ou préparé à partir d'un don de sang ou un mélange de concentrés plaquettaires issus d'aphérèse ou préparés à partir de dons de sang. Par exemple, le mélange comprend entre 2 et 7 concentrés plaquettaires, en particulier entre 3 et 5 concentrés plaquettaires.

Le concentré plaquettaire est soit frais, c'est-à-dire qualifié pour être transfusé à un patient, soit périmé, c'est-à-dire stocké pendant 5 jours ou plus après sa préparation et ne pouvant plus être transfusé à un patient.

La suspension de plaquettes comprend des plaquettes en suspension dans un milieu liquide comprenant du plasma.

Par exemple, le milieu liquide ne comprend que du plasma. Selon un autre exemple, le milieu liquide comprend en outre une solution de conservation des plaquettes, telle que la solution SSP+ (Maco Pharma) ou l'Intersol® (Fenwal).

Dans un exemple particulier, le milieu liquide comprend de 20% à 100%, notamment 30% de plasma et de 0% à 80%, notamment 70% de solution de conservation des plaquettes.

Afin de fournir, c'est-à-dire de mettre à disposition, le lysat plaquettaire à l'état liquide, le procédé de stérilisation comprend la préparation préliminaire du lysat plaquettaire à l'état liquide à partir d'une suspension de plaquettes.

Selon une réalisation particulière, la préparation préliminaire dudit lysat plaquettaire à l'état liquide à partir d'une suspension de plaquettes, comprend les étapes successives suivantes :
- la soumission de ladite suspension de plaquettes à au moins un cycle de congélation / décongélation afin d'obtenir une composition de plaquettes lysées,
- la séparation de ladite composition de plaquettes lysées en une fraction claire de lysat plaquettaire et une fraction de débris cellulaires,
- l'isolement dudit lysat plaquettaire à l'état liquide.

La suspension de plaquettes est soumise à au moins un cycle de congélation / décongélation. En particulier, 2 à 3 cycles de congélation / décongélation sont effectués.

La suspension de plaquettes est d'abord congelée à une température comprise entre -10°C et -80°C, notamment -80°C. La congélation dure au moins 24 heures. Ensuite, la suspension de plaquettes est décongelée à une température allant de 4°C à 37°C, notamment à température ambiante ou à 4°C.

L'étape de congélation / décongélation provoque la destruction des plaquettes avec libération de leur contenu, et notamment de leurs facteurs de croissance endogènes

Après le cycle de congélation / décongélation, on obtient une composition de plaquettes lysées comprenant le contenu des plaquettes, le milieu liquide dans lequel les plaquettes étaient en suspension et les débris cellulaires.

On sépare ensuite ladite composition de plaquettes lysées en une fraction claire de lysat plaquettaire et une fraction de débris cellulaires.

Selon une réalisation particulière, cette séparation est réalisée par centrifugation de ladite composition de plaquettes lysées afin d'obtenir un surnageant de lysat plaquettaire et un sédiment de débris cellulaires.

En variante, la séparation est réalisée par filtration de sorte à obtenir un filtrat de lysat plaquettaire et un retentât de débris cellulaires.

Le lysat plaquettaire à l'état liquide est finalement isolé par extraction. Ce lysat plaquettaire comprend au moins les facteurs de croissance endogènes IGF-1, PDGF-AB, TGF-beta1, EGF, VEGF et bFGF.

La suspension de plaquettes initiale comprenant du plasma, le lysat plaquettaire stérilisé selon l'invention comprend également du plasma, et notamment des constituants plasmatiques tels que le fibrinogène, des globulines, l'albumine, des triglycérides, des interleukines et des interférons.

Une fois le lysat plaquettaire à l'état liquide préparé, le procédé de l'invention comprend la congélation dudit lysat plaquettaire liquide afin d'obtenir un lysat plaquettaire congelé.

La congélation du lysat plaquettaire liquide est réalisée à une température comprise entre -10°C et -80°C, notamment d'environ -80°C.

Le lysat plaquettaire est conditionné dans un récipient résistant à la congélation et notamment dans une poche. Le matériau résistant à la congélation est notamment de l'éthylène acétate de vinyle, le polyéthylène ou un fluoropolymère tel que l'éthylène-propylène fluoré.

Afin de réaliser la stérilisation, le lysat plaquettaire dans son état congelé subit ensuite une irradiation par un rayonnement ionisant. Le lysat plaquettaire est irradié dans un état non lyophilisé, c'est-à-dire qu'il comprend de l'eau.

Selon un mode de réalisation, le rayonnement ionisant est un rayonnement gamma.

Le rayonnement gamma est un rayonnement électromagnétique composé de photons de haute énergie, de l'ordre de 1,6 MeV. Il est par exemple émis par une source de cobalt 60.

Ce rayonnement ionisant provoque la destruction des micro-organismes par rupture de la chaîne d'acide désoxiribonucléique (ADN) ou du brin de l'acide ribonucléique (ARN) permettant ainsi l'inactivation des bactéries et les virus, par exemple.

Cependant, le rayonnement ionisant est susceptible d'endommager les polymères en créant des ions qui se transforment en des radicaux libres actifs et qui, en se recombinant, créent de nouvelles liaisons chimiques permanentes.

Selon l'invention, l'irradiation par rayonnement ionisant est agencée de sorte à conserver au moins 80% de la concentration de l'un au moins des facteurs de croissance endogènes choisi dans le groupe constitué par le TGF-beta1, l'EGF, le PDGF-AB, l'IGF-1 et le VEGF.

En particulier, l'irradiation par rayonnement gamma est agencée de sorte à conserver au moins 80%, particulièrement au moins 90% de la concentration de chacun des facteurs de croissance TGF-beta1, EGF, PDGF-AB, IGF-1 et le VEGF.

Encore plus particulièrement, le lysat plaquettaire irradié selon l'invention conserve au moins 95% de la concentration de chacun des facteurs de croissance TGF-beta1, EGF, PDGF-AB et IGF-1.

Les facteurs de croissance IGF, PDGF, TGF-beta1 et EGF, et VEGF sont les principaux facteurs de croissance présents dans le lysat plaquettaire. En outre, les facteurs de croissance PDGF, bFGF, TGF-beta1 sont d'importance pour la prolifération cellulaire, notamment pour les cellules souches mésenchymateuses. Le maintien de leur concentration dans le lysat plaquettaire stérilisé est donc un indicateur du maintien de l'activité biologique du lysat plaquettaire, notamment en termes de prolifération cellulaire.

Ainsi, le taux d'amplification des cellules souches mésenchymateuses dans une composition nutritive comprenant un milieu de base et un lysat plaquettaire stérilisé selon le procédé de l'invention est conservé à au moins 80%, notamment à au moins 85% par rapport au taux d'amplification des cellules souches mésenchymateuses dans une composition nutritive comprenant un milieu de base et un lysat plaquettaire non stérilisé par le procédé de l'invention.

Par milieu de base, on désigne un milieu destiné à la culture cellulaire tel que le milieu RPMI, MEM, DMEM ou un mélange de ces milieux. Ces milieux de base comprennent essentiellement des sels minéraux, du glucose, des acides aminés, des vitamines et des bases azotées.

Le lysat plaquettaire stérilisé par le procédé de l'invention conserve ainsi une efficacité en termes de prolifération des cellules souches mésenchymateuses.

Selon un mode de réalisation, l'irradiation est réalisée à une dose absorbée comprise dans la plage allant de 20 kGy à 60 kGy, notamment de 35 kGy à 45 kGy.

La dose absorbée est la quantité d'énergie communiquée à la matière par unité de masse.

Par exemple, l'irradiation est effectuée pendant une durée comprise dans la plage allant de 600 secondes à 1800 secondes, préférentiellement de 900 secondes à 1200 secondes, et plus préférentiellement pendant 1075 secondes, avec une source présentant une activité de 1 Mci (3,7x10¹⁹ Bq).

En pratique, l'irradiation par rayonnement ionisant du lysat plaquettaire congelé est réalisée à basse température, le lysat plaquettaire congelé étant placé dans de la glace carbonique.

De façon avantageuse, l'étape d'irradiation est réalisée sur le lysat plaquettaire dans son conditionnement final, notamment dans une poche. La poche est par exemple réalisée en un matériau résistant à l'irradiation par rayonnement ionisant tel que l'éthylène acétate de vinyle.

Dans ces conditions d'irradiation, notamment de dose relativement élevée permettant la destruction des pathogènes et de température très basse, il a été constaté de façon surprenante que la concentration de la plupart des facteurs de croissance contenus dans le lysat plaquettaire restait substantiellement équivalente. Ceci est le cas pour les facteurs de croissance TGF-beta1, EGF, le PDGF-AB, IGF-1, et dans une moindre mesure VEGF.

Seule la concentration en facteur de croissance bFGF subit une baisse plus prononcée, de l'ordre de 40%.

En outre, il est à noter que l'irradiation par rayonnement ionisant est en particulier réalisée sans composé stabilisateur exogène, connu pour réduire les dommages au matériel devant être irradié par rayonnement ionisant. Des exemples de tels composés stabilisateurs sont les antioxydants (acide ascorbique, tocophérol), les capteurs de radicaux libres, certains polysaccharides comme la cellulose ou le chitosane, et certaines protéines comme la gélatine.

Selon une réalisation particulière, le procédé de l'invention comprend en outre une étape de filtration du lysat plaquettaire à l'état liquide au travers d'un filtre de porosité 0,65 µm ou moins, particulièrement 0,45 µm ou moins, et notamment 0,22 µm ou moins.

L'étape de filtration est par exemple réalisée à la fin de la préparation préliminaire du lysat plaquettaire à l'état liquide, avant sa congélation en vue de l'irradiation.

Lorsque le lysat plaquettaire est filtré au travers d'un filtre ayant une porosité de 0,22 µm ou moins, le filtre est dit stérilisant en ce qu'il retient notamment les bactéries de taille supérieure à 0,22 µm.

Dans ce cas, le lysat plaquettaire subit deux procédés de stérilisation : la filtration stérilisante et la stérilisation par rayonnement ionisant, ce qui permet d'élargir le spectre des pathogènes éliminés et/ou inactivés potentiellement présents dans le lysat plaquettaire.

En particulier, le lysat plaquettaire stérilisé selon l'invention est conditionné dans une poche, notamment réalisée en éthylène vinyle acétate.

Selon un autre aspect, l'invention concerne un lysat plaquettaire stérilisé obtenu par le procédé selon le premier aspect de l'invention.

Le lysat plaquettaire préparé selon le procédé de stérilisation de l'invention présente un profil de facteurs de croissance particulier.

Par exemple, Le lysat plaquettaire stérilisé selon l'invention comprend une concentration du facteur de croissance bFGF endogène inférieure à 120 pg/ml, notamment inférieure à 100 pg/ml. La concentration d'IGF-1 endogène est comprise entre 30 et 40 ng/ml. La concentration de PDGF-AB endogène est comprise entre 20 et 45 ng/ml. La concentration de TGF-beta1 endogène est comprise entre 100 et 130 ng/ml. La concentration en EGF endogène est comprise entre 2500 et 3700 ng/ml. La concentration en VEGF endogène est comprise entre 600 et 800 pg/ml, notamment entre 600 et 700 pg/ml.

Le lysat plaquettaire stérilisé selon l'invention comprend également des constituants plasmatiques tels que le fibrinogène, des globulines, l'albumine, des triglycérides, des interleukines et des interférons, dont la quantité varie selon le produit de départ du lysat plaquettaire, notamment selon que la suspension de plaquettes comprend ou non une solution de conservation des plaquettes.

Par exemple, la concentration du fibrinogène endogène dans un lysat plaquettaire stérilisé selon l'invention et préparé à partir d'une suspension de plaquettes comprenant 30% de plasma et 70% d'une solution de conservation des plaquettes est inférieure à 0,4 g/l, soit une perte d'environ 20% par rapport à un lysat plaquettaire non irradié par rayonnement gamma.

Selon un autre exemple, la concentration du fibrinogène endogène dans un lysat plaquettaire stérilisé selon l'invention et préparé à partir d'une suspension de plaquettes comprenant 100% de plasma est inférieure à 1 g/l, particulièrement inférieure à 0,70 g/l et plus particulièrement inférieure à 0,60 g/l, soit une perte en fibrinogène endogène variant de plus de 25% à plus de 45%.

Avantageusement, le lysat plaquettaire de l'invention est dépourvu de substance exogène. De telles substances sont notamment (i) des stabilisateurs tels que des antioxydants (acide ascorbique, tocophérol), des capteurs de radicaux libres, certains polysaccharides comme le cellulose ou le chitosane, certaines protéines comme la gélatine, et des peptide ou dipeptides tels que la glycine ou l'alanylglutamine; (ii) des molécules exogènes capable de lier les facteurs dérivés des plaquettes, telles que l'héparine ou le sulfate de dextrane; (iii) des polymères amphiphiles tels que le polyvinylpyrrolidone ou des dérivés de cellulose.

En outre, le lysat plaquettaire stérilisé selon l'invention présente l'avantage d'avoir des propriétés de coagulation réduites par rapport à un lysat plaquettaire non stérilisé par le procédé de l'invention.

En effet, le procédé classique de préparation du lysat plaquettaire par congélation / décongélation conduit à un lysat plaquettaire comprenant du fibrinogène, protéine soluble présente dans le plasma et impliquée dans la coagulation.

Lorsque le lysat plaquettaire préparé de façon classique est mis en contact avec un milieu de base qui comprend du calcium, le milieu coagule ou "gélifie". Pour éviter cette coagulation, il est nécessaire d'ajouter au milieu de base un anticoagulant de type héparine.

De façon surprenante et particulièrement avantageuse, le lysat plaquettaire obtenu par le procédé de stérilisation ne coagule pas lorsqu'il est ajouté au milieu de culture, notamment lorsque la quantité de lysat plaquettaire ajoutée au milieu de culture est comprise dans la plage allant de 1% à 15%, particulièrement de 2% à 10%, encore plus particulièrement de 2% à 5%. L'ajout d'héparine n'est alors plus nécessaire.

De façon encore plus inattendue, le lysat plaquettaire stérilisé selon l'invention présente des propriétés très particulières en relation avec la prolifération des cellules, notamment des cellules souches mésenchymateuses.

En effet, la complémentation d'un milieu de base avec un lysat plaquettaire stérilisé selon l'invention et du facteur de croissance bFGF exogène induit un effet bénéfique sur la prolifération des cellules souches mésenchymateuses, alors que l'ajout de bFGF exogène à un milieu de base complémenté avec un lysat plaquettaire non irradié par rayonnement ionisant n'a pas d'effet sur cette prolifération.

Il existe donc un effet synergique entre l'ajout de bFGF exogène et l'irradiation par rayonnement ionisant d'un lysat plaquettaire.

Ainsi, selon un troisième aspect, l'invention concerne un procédé pour la culture de cellules, notamment de cellules souches mésenchymateuses comprenant la mise en contact desdites cellules avec une composition nutritive comprenant un milieu de base et un lysat plaquettaire stérilisé selon le deuxième aspect de l'invention.

Les cellules souches mésenchymateuses sont par exemple des cellules souches mésenchymateuses humaines issues de la moelle osseuse ou du sang de cordon ombilical.

Selon une réalisation particulière, la composition nutritive comprend de 2 % à 25%, en particulier de 5% à 15%, et encore plus particulièrement de 8 à 10% de lysat plaquettaire stérilisé selon l'invention.

Notamment, le lysat plaquettaire stérilisé est ajouté extemporanément de façon préliminaire audit milieu de base de sorte à former ladite composition nutritive.

Comme le lysat plaquettaire irradié présente un pouvoir de coagulation réduit, il n'est pas nécessaire d'ajouter à la composition nutritive un anticoagulant de type héparine pour éviter sa coagulation et la maintenir dans un état liquide. Ainsi, selon un mode de réalisation du procédé pour la culture de cellules, la composition nutritive est sous forme liquide et exempte d'anticoagulant.

Avantageusement, le procédé pour la culture de cellules comprend en outre l'ajout extemporané à ladite composition nutritive de bFGF exogène.

En particulier, la concentration de bFGF exogène ajoutée est comprise dans la plage allant de 0,1 ng/ml à 15 ng/ml, notamment de 0,1 à 1,5 ng/ml, par exemple 1 ng/ml.

### Exemple :

### 1. Préparation d'un lysat plaquettaire

Un lot de lysat plaquettaire est préparé comme décrit ci-dessous.

Des concentrés plaquettaires comprenant 70% de solution de conservation Intersol® et 30% de plasma ont été préparés à partir d'un pool de cinq buffy coat et conservés dans une poche de stockage.

Les poches de stockage ont été congelées à -80°C pendant une durée d'environ 24 heures avant d'être décongelées à température ambiante pendant environ 24 heures.

Les poches de stockage décongelées sont ensuite centrifugées à une vitesse de 5000 g pendant 10 minutes de sorte à séparer le surnageant comprenant le lysat plaquettaire du sédiment comprenant les débris cellulaires. Le surnageant de chacune des poches de stockage est transféré dans une poche de mélange de sorte à obtenir un mélange de lysats plaquettaires. Le mélange de lysats plaquettaires est ensuite redistribué dans des petites poches de 50 ml en éthylène acétate de vinyle.

Un autre lot de lysat plaquettaire est préparé de façon similaire, à l'exception que le mélange de lysats plaquettaires est filtré au travers d'un filtre stérilisant de porosité 0,22 µm avant d'être redistribué dans des petites poches en éthylène acétate de vinyle.

Les petites poches sont ensuite congelées à -80°C pour stockage.

### 2. Irradiation des lysats plaquettaires

Les petites poches congelées contenant le lysat plaquettaire sont irradiées par rayonnement gamma à une dose absorbée de 35 kGy ou 45 kGy.

### 3. Dosage des facteurs de croissance

Les facteurs de croissance IGF1, PDGF-AB, TGF-beta1, EGF, VEGF et bFGF sont dosés dans des échantillons des deux lots de lysat plaquettaire (filtré et non filtré), les échantillons ayant été irradiés par rayonnement gamma à 35 kGy ou à 45 kGy ou non irradiés. Trois dosages sont effectués pour chaque échantillon.

Les dosages des facteurs de croissance sont effectués à l'aide des kits commerciaux ELISA Quantikine fournis par Bio-Techne (références DG100 pour l'IGF-1 humain, DHD00C pour le PDGF-AB humain, DB100B pour le TGF-beta1 humain, DEG00 pour l'EGF humain, DVE00 pour le VEGF humain, et DFB50 pour le FRG humain) selon les instructions du fabricant.

La figure 1 représente les moyennes des concentrations en facteurs de croissance IGF-1, PDGF-AB, TGF-beta1, EGF, VEGF et bFGF dans les échantillons non irradiés (C), filtrés ou non, et dans les échantillons irradiés par rayonnement gamma (I) à 35 kGy ou 45 kGy, filtrés ou non.

Les résultats montrent que l'irradiation par rayonnement gamma n'a pas d'effet significatif sur les concentrations des facteurs de croissance IGF-1, PDGF-AB, TGF-beta1 et EGF. L'irradiation par rayonnement gamma a un impact modéré sur la concentration du VEGF (-8%), et plus marqué sur le bFGF (-39%).

### 4. Dosage de composants plasmatiques

Des tests biochimiques ont été réalisés sur un lysat plaquettaire préparé selon l'exemple 1 afin de déterminer la concentration de certains composants.

Le fibrinogène a été dosé selon la méthode de Clauss (dosage chronométrique avec excès de thrombine).

Les D-dimères ont été dosés par immunoturbidimétrique au latex (lecture photométrique).

La vitamine B12 et la vitamine D ont été dosées par une méthode immunoenzymologique micro particulaire par chimiluminescence (CMIA).

Le cholestérol total é été dosé par colorimétrie enzymatique avec le cholestérol estérase/oxydase.

Le sodium et le chlore ont été dosés par potentiomètre indirecte sélective (électrode spécifique)

La colorimétrie a été utilisée pour le dosage des protides totaux (Biuret), de l'albumine (vert de bromocresol), du calcium (Arsenazo III) et du fer sérique (férène sans déprotéinisation).

Les mycoplasmes ont été détectés par culture sur milieu sélective (gélose).

Le tableau ci-dessous montre, qu'à l'exception du fibrinogène, les autres composants testés ne sont pas impactés par l'irradiation gamma à des doses variant de 5 à 55 KGy.

| Lysat plaquettaire | Contrôle | 5 KGy | 15 KGy | 35 KGy | 55 KGy |
|---|---|---|---|---|---|
| Fibrinogène (g/l) | 0,50 | 0,42 | 0,40 | 0,40 | <0,4 |
| D-Dimères (mg/ml) | 0,27 | 0,27 | 0,27 | 0,27 | 0,31 |
| Vitamine B12 (pg/ml) | 144 | 119 | 135 | 134 | 138 |
| Vitamine D (ng/ml) | 5,5 | 5,1 | 4,9 | 5,4 | 5,4 |
| Choléstérol total (g/l) | 0,49 | 0,49 | 0,48 | 0,47 | 0,49 |
| Sodium (mEq/l) | 182 | 182 | 180 | 181 | 181 |
| Chlore (mEq/l) | 78 | 79 | 79 | 79 | 79 |
| Protides totaux (g/l) | 18 | 18 | 18 | 18 | 18 |
| Albumine (g/l) | 12 | 12 | 12 | 12 | 12 |
| Calcium (mg/l) | 31 | 31 | 31 | 31 | 31 |
| Calcium corrigé (mg/l) | 53 | 53 | 53 | 53 | 53 |
| Fer sérique (µg/dl) | 30 | 30 | 31 | 30 | 31 |
| Mycoplasme 10³ UFC/ml) | négatif | négatif | négatif | négatif | négatif |

Les mêmes dosages ont été réalisés sur un lysat plaquettaire obtenu selon procédé de l'exemple 1, à la différence que les concentrés de plaquettes de départ ne comprennent pas de solution de conservation des plaquettes (100% plasma).

| Lysat plaquettaire | Contrôle | 5 KGy | 15 KGy | 35 KGy | 55 KGy |
|---|---|---|---|---|---|
| Fibrinogène (g/l) | 1,34 | 0,92 | 0,69 | 0,51 | 0,4 |

### 4. Prolifération des cellules souches mésenchymateuses

Des cellules souches mésenchymateuses humaines issues de moelle osseuse ont été cultivées (4 000 cellules/cm²) pendant 7 jours dans une composition nutritive comprenant le milieu de base alpha-DMEM complémenté avec 8% de lysat plaquettaire, filtré ou non, irradié par rayonnement gamma à 35 kGy, 45kGy ou non.

Il a d'abord été constaté que l'étape d'irradiation du lysat plaquettaire par rayonnement gamma induit une perte de la propriété de coagulation lorsqu'il est mis en présence du milieu de base, rendant l'ajout d'héparine dans le milieu non nécessaire.

En comparaison, les cellules souches mésenchymateuses humaines issues de la moelle osseuse ont été cultivées dans une composition nutritive comprenant le milieu de base alpha-DMEM complémenté avec 10% de sérum de veau fœtal.

La figure 2 montre les moyennes des facteurs d'amplification des cellules souches mésenchymateuses cultivées avec du lysat plaquettaire non irradié (C), filtré ou non, et irradié par rayonnement gamma à 35 kGy ou à 45 kGy, filtré ou non ; et en comparaison avec des cellules cultivées avec du sérum de veau fœtal et du bFGF.

La moyenne de diminution du facteur d'amplification est de l'ordre de 15%.

### 5. Effet du facteur de croissance bFGF

Le facteur de croissance bFGF est connu pour stimuler la prolifération de nombreux types cellulaires. Cependant, lorsque la culture des cellules souches mésenchymateuses est réalisée avec du lysat plaquettaire en remplacement du sérum de veau fœtal, l'effet du bFGF exogène sur le taux d'amplification cellulaire est négligeable.

L'effet sur la prolifération de cellules souches mésenchymateuses de l'addition de bFGF exogène (1 ng/ml) à un milieu de base supplémenté avec du lysat plaquettaire irradié (8%) par rayonnement gamma (I) ou non irradié (C) a été testé.

Pour comparaison, l'effet sur la prolifération de cellules souches mésenchymateuses de l'addition du bFGF exogène (1 ng/ml) à un milieu de base complémenté avec du sérum de veau fœtal (10%) de grade de recherche (SVF (RG)) ou de grade clinique (SVF (CG) a également été testé.

La figure 3 montre la moyenne des taux d'amplification obtenus. Les résultats confirment l'effet du bFGF dans les cultures en sérum de veau fœtal, et l'absence d'effet de bFGF exogène dans les cultures en lysat plaquettaire non irradié.

Par contre, en ajoutant 60 pg/ml ou 150 pg/ml à un lysat plaquettaire irradié par rayonnement gamma, le taux d'amplification redevient équivalent à celui obtenu avec du lysat plaquettaire non irradié. Et l'ajout de 1 ng/ml de bFGF exogène montre un meilleur taux d'amplification.

### 6. Phénotype

Il a été vérifié que l'utilisation d'un lysat plaquettaire, combiné ou non à du bFGF exogène, pour la culture de cellules souches mésenchymateuses ne modifie pas le profil d'expression de ces cellules, celles-ci restant positives pour les marqueurs CD13, CD44, CD73, CD90 et CD105 et négatives pour les marqueurs CD34, CD45 et HLA-DR. Les cellules sont également négatives pour les marqueurs CD40, CD80 et CD86.

## Revendications

1. Procédé de stérilisation d'un lysat plaquettaire à l'état liquide comprenant au moins les facteurs de croissance endogènes TGF-beta1, EGF, PDGF-AB, IGF-1, VEGF et bFGF, ledit procédé comprenant la congélation dudit lysat plaquettaire liquide afin d'obtenir un lysat plaquettaire congelé, ledit procédé étant **caractérisé en ce qu'**il comprend en outre l'irradiation par un rayonnement ionisant dudit lysat plaquettaire congelé afin d'obtenir un lysat plaquettaire stérilisé, ladite irradiation étant agencée de sorte à conserver au moins 80% de la concentration de l'un au moins des facteurs de croissance endogènes choisi dans le groupe constitué par le TGF-beta1, l'EGF, le PDGF-AB, l'IGF-1 et le VEGF.

2. Procédé selon la revendication 1, **caractérisé en ce que** l'irradiation est agencée de sorte à conserver au moins 80% de la concentration de chacun des facteurs de croissance TGF-beta1, EGF, PDGF-AB, IGF-1 et le VEGF.

3. Procédé selon l'une des revendications 1 ou 2, **caractérisé en ce qu'**il comprend la préparation préliminaire du lysat plaquettaire à l'état liquide à partir d'une suspension de plaquettes.

4. Procédé selon la revendication 3, **caractérisé en ce que** la préparation préliminaire dudit lysat plaquettaire à l'état liquide à partir d'une suspension de plaquettes comprend les étapes successives suivantes :
- la soumission de ladite suspension de plaquettes à au moins un cycle de congélation / décongélation afin d'obtenir une composition de plaquettes lysées,
- la séparation de ladite composition de plaquettes lysées en une fraction claire de lysat plaquettaire et une fraction de débris cellulaires,
- l'isolement dudit lysat plaquettaire à l'état liquide.

5. Procédé selon l'une quelconque des revendications 1 à 4, **caractérisé en ce qu'**il comprend une étape de filtration dudit lysat plaquettaire à l'état liquide au travers d'un filtre de porosité 0,65 µm ou moins, particulièrement 0,45 µm ou moins, et notamment 0,22 µm ou moins.

6. Procédé selon l'une quelconque des revendications 3 à 5, **caractérisé en ce que** ladite suspension de plaquettes comprend des plaquettes en suspension dans un milieu liquide comprenant du plasma.

7. Procédé selon la revendication 6, **caractérisé en ce que** ledit milieu liquide comprenant du plasma comprend en outre une solution de conservation des plaquettes.

8. Procédé selon l'une quelconque des revendications 1 à 7, **caractérisé en ce que** la congélation du lysat plaquettaire liquide est réalisée à une température d'environ -80°C.

9. Procédé selon l'une quelconque des revendications 1 à 8, **caractérisé en ce que** le rayonnement ionisant est un rayonnement gamma.

10. Procédé selon l'une quelconque des revendications 1 à 9, **caractérisé en ce que** l'irradiation est réalisée à une dose absorbée comprise dans la plage allant de 20 kGy à 60 kGy, notamment de 35 kGy à 45 kGy.

11. Lysat plaquettaire stérilisé obtenu par le procédé selon l'une quelconque des revendications 1 à 10.

12. Lysat plaquettaire stérilisé selon la revendication 11, **caractérisé en ce qu'**il comprend une concentration du facteur de croissance bFGF endogène inférieure à 120 pg/ml.

13. Lysat plaquettaire stérilisé selon l'une des revendications 11 ou 12, **caractérisé en ce qu'**il comprend une concentration du facteur de croissance VEGF endogène comprise entre 600 et 700 pg/ml.

14. Lysat plaquettaire stérilisé selon l'une quelconque des revendications 11 à 13, **caractérisé en ce qu'**il est conditionné dans une poche.

15. Procédé pour la culture de cellules, notamment de cellules souches mésenchymateuses, comprenant la mise en contact desdites cellules avec une composition nutritive comprenant un milieu de base et un lysat plaquettaire stérilisé selon l'une quelconque des revendications 11 à 14.

16. Procédé selon la revendication 15, **caractérisé en ce qu'**il comprend l'étape préliminaire d'ajout extemporané dudit lysat plaquettaire stérilisé audit milieu de base de sorte à former ladite composition nutritive.

17. Procédé selon l'une des revendications 15 ou 16, **caractérisé en ce qu'**il comprend en outre l'ajout extemporané à ladite composition nutritive de bFGF exogène.

18. Procédé selon la revendication 17, **caractérisé en ce que** la concentration de bFGF exogène ajoutée est comprise dans la plage allant de 0,1 ng/ml à 15 ng/ml, notamment 1 ng/ml.

19. Procédé selon l'une quelconque des revendications 15 à 18, **caractérisé en ce que** la composition nutritive est sous forme liquide et exempte d'anticoagulant.
